# EUROPEAN PATENT APPLICATION

(11) **EP 3 095 868 A1**
(43) Date of publication of application: **23.11.2016**
(21) Application number: 15168106.1
(22) Date of filing: 19.05.2015
(51) Int. Cl.: C12N 15/52, C12R 1/00

(54) **METHIONINE PRODUCTION**

(71) Applicant: Evonik Degussa GmbH, 45128 Essen (DE)
(72) Inventor: Haas, Thomas, 48161 Münster (DE); Bülter, Thomas, 47279 Duisburg (DE); Thiessenhusen, Anja, 48147 Münster (DE)

(57) **Abstract**

The present invention relates to a microbial cell capable of producing at least one acetylhomoserine from 4-acetyl-butyric acid and/or ester thereof, wherein the cell is genetically modified to comprise
- at least a first genetic mutation that increases the expression relative to the wild type cell of an enzyme E₁ capable of catalysing the hydroxylation of 4-acetyl-butyric acid and/or ester thereof to an alpha-hydroxy acetyl butanone, and
- at least a second genetic mutation that increases the expression relative to the wild type cell of enzyme E₂ capable of catalysing the amination of the alpha-hydroxy acetyl butanone to the acetylhomoserine.

## Description

### FIELD OF THE INVENTION

The present invention relates to a biotechnological method for producing methionine. In particular, the method may use carbon as the starting material.

### BACKGROUND OF THE INVENTION

Amino acids are especially useful as additives in animal feed and as nutritional supplements for human beings. They can also be used in infusion solutions and may function as synthetic intermediates for the manufacture of pharmaceuticals and agricultural chemicals. Compounds such as cysteine, homocysteine, methionine and S-adenosylmethionine are usually industrially produced to be used as food or feed additives and also in pharmaceuticals. In particular, methionine, an essential amino acid, which cannot be synthesized by animals, plays an important role in many body functions. D,L-methionine is presently being produced by chemical synthesis from hydrogen cyanide, acrolein and methyl mercaptan. These petroleum based starting materials such as acrolein and methyl mercaptan are obtained by cracking gasoline or petroleum which is bad for the environment. Also, since the costs for these starting materials will be linked to the price of petroleum, with the expected increase in petroleum prices in the future, prices of methionine will also increase relative to the increase in the petroleum prices.

There are several chemical means of producing methionine. In one example, 3-methylthiopropanal is used as a raw material with hydrocyanic acid in the presence of a base. The reaction results in ammonium carbonate, which is then hydrolysed. In this method, carbon dioxide is introduced into the reaction liquid after hydrolysis, whereby crystallization occurs and methionine is separated as a crystal. Carbon dioxide and hydrogen are used as raw materials for producing methionine using this method. However, a large amount of hydrogen is left over, making this method inefficient.

With the increasing methionine demand, thus microbial production of methionine is always an attractive alternative.

The pathway for L-methionine synthesis is well known in many microorganisms (Figge RM (2006)). *E. coli* and C. *glutamicum* methionine producer strains have also been described in patent applications WO2005/111202, WO2007/077041, WO2007/012078 and WO2007/135188. Methionine produced by fermentation needs to be purified from the fermentation broth. Cost-efficient purification of methionine relies on producer strains and production processes that minimize the amount of by-products in the fermentation broth. Further, most of these biotechnological methods of producing methionine use nutrients including, but not limited to, carbohydrate sources, e.g., sugars, such as glucose, fructose, or sucrose, hydrolyzed starch, nitrogen sources, e.g., ammonia, and sulfur sources e.g., sulfate and/or thiosulfate, together with other necessary or supplemental media components as a starting material. This process would yield L-methionine and biomass as a by-product with no toxic dangerous, flammable, unstable, noxious starting materials. However, this is an expensive raw material and the yield too low to consider this method commercially viable.

Accordingly, there is a need in the art for a cheaper and more efficient biotechnological means of producing methionine.

### DESCRIPTON OF THE INVENTION

The present invention provides a cell that may be capable of producing homoserines that are essential in the production of methionine. In particular, the cell may be genetically modified to express at least an enzyme E₁ capable of catalysing the hydroxylation of 4-acetyl-butyric acid and/or ester thereof to an alpha-hydroxy acetyl butanoate. The cell may also be used in a method of converting 4-acetyl-butyric acid and/or ester thereof to an alpha-hydroxy acetyl butanoate. More in particular, the cell may be genetically modified to comprise other specific enzymes that are capable of catalysing the conversion of a simple carbon source to acetyl-butyric acid and/or ester and also the conversion of alpha-hydroxy acetyl butanoate to a homoserine. The ability of enzyme E₁ to catalyse the hydroxylation of 4-acetyl-butyric acid and/or ester thereof to an alpha-hydroxy acetyl butanoate, allows for the biotechnological production of homoserine to be more efficient and cost effective. This is because, the carbon source may include CO and/or CO₂ allowing the feedstock to be cheap and carbon yield to be close to 100%.

According to one aspect of the present invention, there is provided a microbial cell capable of producing at least one acetylhomoserine from 4-acetyl-butyric acid and/or ester thereof, wherein the cell is genetically modified to comprise
- at least a first genetic mutation that increases the expression relative to the wild type cell of an enzyme E₁ capable of catalysing the hydroxylation of 4-acetyl-butyric acid and/or ester thereof to an alpha-hydroxy acetyl butanone, and
- at least a second genetic mutation that increases the expression relative to the wild type cell of enzyme E₂ capable of catalysing the amination of the alpha-hydroxy acetyl butanone to the acetylhomoserine.

The starting material for the hydroxylation may be 4-acetyl-butyric acid and/or ester thereof. In particular, the ester of 4-acetyl-butyric acid may be produced in the presence of at least one WS/DGAT enzyme found in the cell according to any aspect of the present invention and/or other esterification processes known in the art.

In one example, the method of esterification may be a chemical process. Any chemical reaction known in the art may be used to convert 4-acetyl-butyric acid to 4- acetoxybutanoate. More in particular, the method involves reacting 4-acetyl-butyric acid with an alcohol. 4-acetyl-butyric acid may be contacted with at least one alcohol to produce a 4- acetoxybutanoate. In particular, the alcohol may be methanol, ethanol, isopropyl alcohol, and/or butyl alcohol. In particular, the alcohol may be selected from the group consisting of methanol, ethanol, isopropyl alcohol, and/or butyl alcohol to produce methyl, ethyl, propyl or butyl butanoate respectively. This reaction may take place in the presence of at least one catalyst. The catalyst is usually an acidic catalyst such as s sulphonic acid, a base such as an alkali hydroxide or an alkali alcoholate, a metal oxide or a metal alkylate. In particular, the catalyst may be ZrOCl₂·8H₂O.

In one example, the 4-acetyl-butyric acid reacts with ethanol in the presence of ZrOCl₂·8H₂O to produce ethyl 4-acetoxybutanoate.

In another example, the method of esterification may be a biochemical process where at least one enzyme may be involved in catalysing the esterification process. The enzyme for esterification may be selected from the group consisting of a thioesterase enzyme, an acyl-CoA synthetase enzyme, an ester synthase enzyme and a lipase. At least one of these enzymes may be expressed in the cell according to any aspect of the present invention. In one example, the acetogenic bacteria and/or hydrogen oxidising bacteria may overexpress an esterification enzyme that may be capable of esterification of the 4-acetyl-butyric acid. In another example, a further cell expressing an esterification enzyme may be included to esterify the 4-acetyl-butyric acid to 4-acetoxybutanoate.

In particular, the enzymes in the cell according to any aspect of the present invention may comprise at least one of the following enzymes: cytochrome P450 BM3 (CYP102A1) (E₁) and a leucine dehydrogenase or alpha transaminase (E₂). More in particular, the cell according to any aspect of the present invention may be capable of increased expression relative to the wild type cell of cytochrome P450 BM3 (CYP102A1) (E₁) and a leucine dehydrogenase (E₂).

The cell according to any aspect of the present invention, allows for a two-step process to be carried out in a single pot with no product purification/separation of the polar substrate intermediates (α-hydroxy acid, α-keto acid) required between the stages.

The cell according to any aspect of the present invention further comprises at least a third genetic mutation that increases the expression relative to the wild type cell of at least one enzyme (E₅) capable of cofactor regeneration. In particular, E₅ may be capable of NAD(P)H regeneration. E₅ may be any enzyme that may be capable of NAD(P)H regeneration. In particular, E₅ may be a dehydrogenase/ oxidoreductase which uses NAD(P) as electron acceptor (EC 1.1.1.X). More in particular, E₅ may be any enzyme with KEGG no. EC 1.1.1.X in the Brenda database as of 24^{th} February 2014. For example, E₅ may be selected from the group consisting of alcohol dehydrogenase, glycerol phosphate dehydrogenase, histidinol dehydrogenase, shikimate dehydrogenase, lactate dehydrogenase, 3-hydroxyaryl-CoA dehydrogenase, malate dehydrogenase, isocitrate dehydrogenase, glucose-6-phosphate dehydrogenase, formate dehydrogenase, horse liver alcohol dehydrogenase, glucose dehydrogenase, amino acid dehydrogenase, sorbitol dehydrogenase, 20-β-hydroxysteroid dehydrogenase and formaldehyde dehydrogenase. Even more in particular, enzyme (E₅) may be selected from the group consisting of glucose dehydrogenase (E₅ₐ) (EC 1.1.99.10), phosphite dehydrogenase (E_{5b}) (EC 1.20.1.1) and formate dehydrogenase (E_{5c}) (EC 1.2.1.43) where glucose, phosphite and formate are used as reducing agents respectively. The presence of enzyme (E₅) allows for cofactor regeneration that enables the process of producing alpha-hydroxy acetyl butanone from 4-acetyl-butyric acid and/or ester thereof to be self-sustaining. No external energy would thus have to be introduced into the system of producing alpha-hydroxy acetyl butanone. Accordingly, the cell according to any aspect of the present invention may be able to generate at least one alpha-hydroxy acetyl butanone from a 4-acetyl-butyric acid and/or ester thereof in the presence of at least enzymes E₁, and/or E₅ without any external energy source needed.

In one example, the glucose dehydrogenase (E₅ₐ) may be NADP+-specific glucose dehydrogenase. The organism that serves as the source of glucose dehydrogenase (E₅ₐ) is not subject to limitation, and may be a microorganism or a higher organism, and microorganisms such as bacteria, fungi, and yeast are suitable. For example, a microorganism of the genus *Bacillus,* in particular *Bacillus megaterium,* may be the source. In another example, the source may be a microorganism belonging to the genus *Cryptococcus,* the genus *Gluconobacter,* or the genus *Saccharomyces.* In particular, a microorganism belonging to the genus *Cryptococcus* may be selected, more in particular, the microorganism may be selected from the group consisting of *Cryptococcus albi dus, Cryptococcus humicolus, Cryptococus terreus,* and *Cryptococcus uniguttulatus.*

In another example, enzyme E₅ may be phosphite dehydrogenase (E_{5b}) or formate dehydrogenase (E_{5c}). The organism that serves as the source of phosphite dehydrogenase (E_{5b}) or formate dehydrogenase (E_{5c}) may not be subject to limitation, and may be a microorganism or a higher organism, and microorganisms such as bacteria, fungi, and yeast are suitable

In one example, the glucose dehydrogenase (E₅ₐ) may be NADP+-specific glucose dehydrogenase. The organism that serves as the source of glucose dehydrogenase (E₅ₐ) is not subject to limitation, and may be a microorganism or a higher organism, and microorganisms such as bacteria, fungi, and yeast are suitable. For example, a microorganism of the genus *Bacillus,* in particular *Bacillus megaterium,* may be the source. In another example, the source may be a microorganism belonging to the genus *Cryptococcus,* the genus *Gluconobacter,* or the genus *Saccharomyces.* In particular, a microorganism belonging to the genus *Cryptococcus* may be selected, more in particular, the microorganism may be selected from the group consisting of *Cryptococcus albi dus, Cryptococcus humicolus, Cryptococus terreus,* and *Cryptococcus uniguttulatus.*

In another example, enzyme E₅ may be phosphite dehydrogenase (E_{5b}) or formate dehydrogenase (E_{5c}). The organism that serves as the source of phosphite dehydrogenase (E_{5b}) or formate dehydrogenase (E_{5c}) may not be subject to limitation, and may be a microorganism or a higher organism, and microorganisms such as bacteria, fungi, and yeast are suitable

Another advantage of the cell according to any aspect of the present invention lies in the activity of enzymes E₁ and E₅ being redox-neutral, which saves significant cost of redox equivalents and the reaction equilibrium may be completely shifted towards amino acid production. In particular, enzymes E₁ and E₅ allow for recycling of NAD(P)H co-factor.

The cell according to any aspect of the present invention may refer to a wide range of microbial cells. In particular, the cell may be a prokaryotic or a lower eukaryotic cell selected from the group consisting of *Pseudomonas, Corynebacterium, Bacillus* and *Escherichia.* In one example, the cell may be *Escherichia coli.* In another example, the cell may be a lower eukaryote, such as a fungus from the group comprising *Saccharomyces, Candida, Pichia, Schizosaccharomyces* and *Yarrowia,* particularly, *Saccharomyces cerevisiae.* The cell may be an isolated cell, in other words a pure culture of a single strain, or may comprise a mixture of at least two strains. Biotechnologically relevant cells are commercially available, for example from the American Type Culture Collection (ATCC) or the German Collection of Microorganisms and Cell Cultures (DSMZ). Particles for keeping and modifying cells are available from the prior art, for example Sambrook/Fritsch/Maniatis (1989).

The cell may be genetically modified to increase the expression relative to the wild type cell of enzyme E₁ capable of catalysing the hydroxylation of the 4-acetyl-butyric acid and/or ester thereof to an alpha-hydroxy acetyl butanone, and/or enzyme E₂ capable of catalysing the oxidation of the alpha-hydroxy acetyl butanone to the acetylhomoserine. E₁ may be cytochrome P450 BM3 (CYP102A1) (E₁), and/or E₂ may be an amino acid dehydrogenase.

The phrase "wild type" as used herein in conjunction with a cell or microorganism may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

Any of the enzymes used according to any aspect of the present invention, may be an isolated enzyme. In particular, the enzymes used according to any aspect of the present invention may be used in an active state and in the presence of all cofactors, substrates, auxiliary and/or activating polypeptides or factors essential for its activity. The term "isolated", as used herein, means that the enzyme of interest is enriched compared to the cell in which it occurs naturally. The enzyme may be enriched by SDS polyacrylamide electrophoresis and/or activity assays. For example, the enzyme of interest may constitute more than 5, 10, 20, 50, 75, 80, 85, 90, 95 or 99 percent of all the polypeptides present in the preparation as judged by visual inspection of a polyacrylamide gel following staining with Coomassie blue dye.

The cell and/or enzyme used according to any aspect of the present invention may be recombinant. The term "recombinant" as used herein, refers to a molecule or is encoded by such a molecule, particularly a polypeptide or nucleic acid that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

Whether or not a nucleic acid molecule, polypeptide, more specifically an enzyme used according to any aspect of the present invention, is recombinant or not has not necessarily implications for the level of its expression. However, in one example one or more recombinant nucleic acid molecules, polypeptides or enzymes used according to any aspect of the present invention may be overexpressed. The term "overexpressed", as used herein, means that the respective polypeptide encoded or expressed is expressed at a level higher or at higher activity than would normally be found in the cell under identical conditions in the absence of genetic modifications carried out to increase the expression, for example in the respective wild type cell. The person skilled in the art is familiar with numerous ways to bring about overexpression. For example, the nucleic acid molecule to be overexpressed or encoding the polypeptide or enzyme to be overexpressed may be placed under the control of a strong inducible promoter such as the lac promoter. The state of the art describes standard plasmids that may be used for this purpose, for example the pET system of vectors exemplified by pET-3a (commercially available from Novagen). Whether or not a nucleic acid or polypeptide is overexpressed may be determined by way of quantitative PCR reaction in the case of a nucleic acid molecule, SDS polyacrylamide electrophoreses, Western blotting or comparative activity assays in the case of a polypeptide. Genetic modifications may be directed to transcriptional, translational, and/or post-translational modifications that result in a change of enzyme activity and/or selectivity under selected and/or identified culture conditions. Thus, in various examples of the present invention, to function more efficiently, a microorganism may comprise one or more gene deletions. Gene deletions may be accomplished by mutational gene deletion approaches, and/or starting with a mutant strain having reduced or no expression of one or more of these enzymes, and/or other methods known to those skilled in the art.

E₁, the cytochrome P450 BM3 (CYP102A1), may lead to double oxidation of 4-acetyl-butyric acid and/or ester thereof to form the equivalent ketone, alpha-hydroxy acetyl butanone. In one example, E₁ may comprise an amino acid sequence that has 50, 55, 60, 65, 70, 75, 80, 85, 90, 95 or 100% sequence identity to SEQ ID NO:1. More in particular, E₁ may comprise mutations in at least one amino acid. In one example, E₁ may comprise point mutations at least on amino acids at positions 47, 51 and/or 401. In particular, the sequence of E₁ may comprise point mutations R47S, Y51W, I401M.

In one example, O₂ may be used as an oxidant.

In another example, the 4-acetyl-butyric acid may first be esterified by simple methods known in the art to form 4-acetyl-butanoate. 4-acetyl-butanoate (also known as 4-acetyl-butyrate) may be used as the substrate for hydroxylation to form the respective ketone, alpha-hydroxy acetyl butanone.

In one example, the oxidation of 4-acetyl-butyric acid and/or ester thereof to form the alpha-hydroxy acetyl butanone is carried out in 2 steps and catalysed by two different enzymes. In this example, E₃ and E₄ carry out the activity of E₁. In particular, E₃ may catalyse the hydroxylation of 4-acetyl-butyric acid and/or ester thereof to an alpha-hydroxy acetyl butanoate, and E₄ may catalyse the oxidation of the alpha-hydroxy acetyl butanoate and/or ester to the alpha-hydroxy acetyl butanone. In this example, therefore the cell according to any aspect of the present invention may be genetically modified to only increase the expression relative to the wild type cell of two enzymes: E₃ and E₄.

According to another aspect of the present invention, there is provided a microbial cell capable of producing at least one acetylhomoserine from 4-acetyl-butyric acid and/or ester thereof, wherein the cell is genetically modified to comprise
- at least a first genetic mutation that increases the expression relative to the wild type cell of an enzyme E₃ capable of catalysing the hydroxylation of 4-acetyl-butyric acid and/or ester thereof to an alpha-hydroxy acetyl butanoate,
- at least a second genetic mutation that increases the expression relative to the wild type cell of an enzyme E₄ capable of catalysing the oxidation of the alpha-hydroxy acetyl butanoate and/or ester to an alpha-hydroxy acetyl butanone, and
- at least a further genetic mutation that increases the expression relative to the wild type cell of enzyme E₂ capable of catalysing the amination of the alpha-hydroxy acetyl butanone to the acetylhomoserine.

E₃ may be any alpha monooxygenase used according to any aspect of the present invention may be an enzyme capable of catalyzing the alpha-hydroxylation of 4-acetyl-butyric acid and/or ester thereof. In particular, E₃ may be an oxidase selected from the group consisting of rubredoxin-dependent alkane oxidases (EC 1.14.15.3), cytochrome P450 alkane oxidases (EC 1.14.14.1), xylene monooxygenases (EC 1.14.15.-), methane monoxygenases (EC 1.14.13.25), lipoxygenase (EC 1.13.11.-), hydratase (EC 4.2.1.-), 12-hydroxylase (EC 1.14.13.-), diol synthase (EC 1.13.11.-and EC 1.13.12.-), and variants thereof. In particular, the monooxygenase may be capable of oxidising the 4-acetyl-butyric acid and/or ester thereof to an alpha-hydroxy acetyl butanoate. In particular, the monooxygenase may be a cytochrome P450 enzyme. More in particular, the cytochrome P450 enzyme may be P450_{Cla} or P450_{BSB} (EC 1.11.2.4).

In one example, E₁ and/or E₃ may be a cytochrome P450 enzyme that comes in contact with H₂O₂ according to any aspect of the present invention. The application of H₂O₂ and stability of the P450 enzyme in presence of peroxide may be improved with use of an in-situ H₂O₂ producing system. An example of the in-situ H₂O₂ producing system may involve the controlled formation of peroxide in the reaction vessel. In particular, controlled formation of peroxide in the reaction vessel may be carried out using a sensor-controlled electrochemical generation of H₂O₂ (Getrey et al., 2014 and Kreig et al., 2011). In another example, E₁ and/or E₃ may be capable of catalysing O₂-dependent hydroxylation where O₂ may be the sole oxidant.

According to any aspect of the present invention, the dehydrogenase (E₄) may be capable of catalysing the conversion/ oxidation of alpha-hydroxy acetyl butanoate and/or ester to an alpha-hydroxy acetyl butanone. In particular, E₄ may be an alpha-hydroxyacid dehydrogenase (EC 1.1.3.15). More in particular, E₄ may be 2-hydroxyisocaproate dehydrogenase (EC 1.1.1.-) and/or lactate dehydrogenase (EC 1.1.1.28). Even more in particular, the dehydrogenase used according to any aspect of the present invention may comprise an amino acid sequence that has 50, 55, 60, 65, 70, 75, 80, 85, 90, 95 or 100% sequence identity to SEQ ID NO: 3 and/or SEQ ID NO: 5. In another example, E₂ may be an alpha-hydroxy acid oxidase (EC 1.1.3.15), which can catalyse the reaction using O₂ as oxidant (producing H₂O₂). This would be advantageous for the oxidation of α-hydroxyacids 1a-d (scheme 1), as it is irreversible and produces H₂O₂ (Malca et al., 2011) using P₄₅₀ catalysts (Scheme 1). In particular, E₄ may be an FMN-dependent 2-hydroxyacid oxidase (EC 1.1.3.15). More in particular, E₄ may be selected from the group consisting of lactate oxidase (EC 1.1.3.2), (S)-2-hydroxy-acid oxidase (EC 1.1.3.15) and D- or L-mandelate oxidase [EC 1.1.3.X].

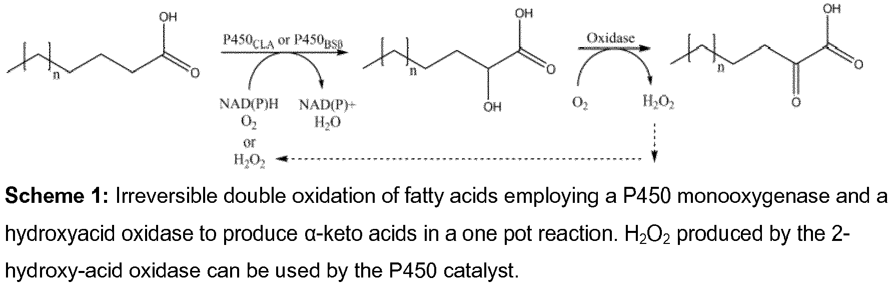

In another example, E₄ may be a monooxygenase where O₂ may be used as an oxidant. In another example, E₄ may be a lactate monooxygenase (EC 1.13.12.4) or a P450 monooxygenase which may be capable of oxidising alpha-hydroxy acetyl butanoate and/or ester to an alpha-hydroxy acetyl butanone.

The teachings of the present invention may not only be carried out using biological macromolecules having the exact amino acid or nucleic acid sequences referred to in this application explicitly, for example by name or accession number, or implicitly, but also using variants of such sequences. The term "variant", as used herein, comprises amino acid or nucleic acid sequences, respectively, that are at least 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner to the effect that the biological activity of the reference sequence or a molecule derived therefrom is preserved. The state of the art comprises algorithms that may be used to align two given nucleic acid or amino acid sequences and to calculate the degree of identity, see Arthur Lesk (2008), Thompson *et* al., 1994, and Katoh *et* al., 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In one example, the term "variant", with regard to amino acid sequence, comprises, in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In one example, the term "variant" of an amino acid sequence or nucleic acid sequence comprises, in addition to the above degree of sequence identity, any active portion and/or fragment of the amino acid sequence or nucleic acid sequence, respectively, or any nucleic acid sequence encoding an active portion and/or fragment of an amino acid sequence. The term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease may be capable of hydrolysing peptide bonds in polypeptides. The phrase "retains at least some of its essential biological activity", as used herein, means that the amino acid sequence in question has a biological activity exceeding and distinct from the background activity and the kinetic parameters characterising said activity, more specifically k_{cat} and K_{M}, are preferably within 3, 2, or 1 order of magnitude of the values displayed by the reference molecule with respect to a specific substrate. Similarly, the term "variant" of a nucleic acid comprises nucleic acids the complementary strand of which hybridises, preferably under stringent conditions, to the reference or wild type nucleic acid. A skilled person would be able to easily determine the monooxygenases that will be capable of making hydroxyl fatty acids.

Stringency of hybridisation reactions is readily determinable by one ordinary skilled in the art, and generally is an empirical calculation dependent on probe length, washing temperature and salt concentration. In general, longer probes require higher temperatures for proper annealing, while shorter probes need lower temperatures. Hybridisation generally depends on the ability of denatured DNA to reanneal to complementary strands when present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995). The person skilled in the art may follow the instructions given in the manual "The DIG System Users Guide for Filter Hybridization", Boehringer Mannheim GmbH, Mannheim, Germany, 1993 and in Liebl *et* al., 1991 on how to identify DNA sequences by means of hybridisation. In one example, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example by lowering the concentration of salt to 2 x SSC or, optionally and subsequently, to 0,5 x SSC, while the temperature is, in order of increasing preference, approximately 50 °C - 68 °C, approximately 52 °C - 68 °C, approximately 54 °C - 68 °C, approximately 56 °C - 68 °C, approximately 58 °C - 68 °C, approximately 60 °C - 68 °C, approximately 62 °C - 68 °C, approximately 64 °C - 68 °C, approximately 66 °C - 68 °C. In a particularly preferred embodiment, the temperature is approximately 64 °C - 68 °C or approximately 66 °C - 68 °C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. The term "homologue" of a nucleic acid sequence, as used herein, refers to any nucleic acid sequence that encodes the same amino acid sequence as the reference nucleic acid sequence, in line with the degeneracy of the genetic code.

E₂ used according to any aspect of the present invention may be an amino acid dehydrogenase. In particular, the amino acid dehydrogenase may be capable of catalysing the conversion of alpha-hydroxy acetyl butanone, ammonia and NADH to acetylhomoserine, water and NAD+. The amino acid dehydrogenase may be an alanine dehydrogenase, i.e. an enzyme capable of catalysing the conversion of pyruvate, ammonia and NADH to L-alanine, water and NAD+. Examples of suitable amino acid dehydrogenases comprise alanine dehydrogenases from *Bacillus subtilis* (accession number: L20916), *Rhizobium leguminosarum* (accession number: CP001622), *Vibrio proteolytikus* (accession number: AF070716), *Mycobacterium tuberculosis* (accession number: X63069) and *Enterobacter aerogenes* (accession number AB013821 In another example, E₂ may be leucine dehydrogenase (EC 1.4.1.9) capable of deamination of L-norleucine and L-leucine. More in particular, the leucine dehydrogenase used according to any aspect of the present invention may comprise an amino acid sequence that has 50, 55, 60, 65, 70, 75, 80, 85, 90, 95 or 100% sequence identity to SEQ ID NO:4. In another example, E₂ may be an ω-transaminase. The ω-transaminase may be selected from any of them as described in Hwang and Kim (2004), Kaulmann et al. (2007), and Yun et al. (2005).

A skilled person would be capable of easily measuring the activity of each of the enzymes E₁, E₂, E₃ and E₄. For example, to determine if the expression of E₁ and/or E₃ is increased in a cell, a skilled person may use the assay disclosed in Malca S.H, 2011. For example, to determine if the expression of E₄ is increased in a cell, a skilled person may use the assay disclosed in H. Schütte et al, 1984 and Hummel W., et al., 1985. The expression of E₂ in a cell, whether it is increased or decreased, may be measured using the assay disclosed at least in Abrahamson M.J., 2012. A skilled person would easily be able to identify other well-known methods in the art that may be used for measuring the expression of the enzymes used in the cell of the present invention.

In one example, a source of amine may be added to the cell according to any aspect of the present invention to produce acetylhomoserine from 4-acetyl-butyric acid and/or ester thereof. In particular, the source of amines may be any ammonium salt known in the art. More in particular, the amine may be selected from the group consisting of ammonium formate, ammonium chloride, ammonium sulphate and the like. In one example, when E₂ may be an alpha transaminase, the source of amine may be alanine.

The cell according to any aspect of the present invention may have reduced capacity of fatty acid degradation by beta-oxidation relative to the wild type cell. In particular, the reduced fatty acid degradation activity compared to the wild type cell may be a result of decreased expression relative to the wild type cell of at least one enzyme selected from the group consisting of acyl-CoA dehydrogenase (FadE) (E₆) (EC:1.3.99.-), enoyl-CoA hydratase (FadB) (E₇) (EC 4.2.1.17), (R)-3-hydroxyacyl-CoA dehydrogenase (FadB) (E₈) (EC 1.1.1.35) and 3-ketoacyl-CoA thiolase (FadA) (E₉) (EC:2.3.1.16).

The term "having a reduced fatty acid degradation capacity", as used herein, means that the respective cell degrades fatty acids, in particular those taken up from the environment, at a lower rate than a comparable cell or wild type cell having normal fatty acid degradation capacity would under identical conditions. In one example, the fatty acid degradation of such a cell is lower on account of deletion, inhibition or inactivation of at least one gene encoding an enzyme involved in the β-oxidation pathway. In one example, at least one enzyme involved in the β-oxidation pathway has lost, in order of increasing preference, 5, 10, 20, 40, 50, 75, 90 or 99 % activity relative to the activity of the same enzyme under comparable conditions in the respective wild type microorganism. The person skilled in the art may be familiar with various techniques that may be used to delete a gene encoding an enzyme or reduce the activity of such an enzyme in a cell, for example by exposition of cells to radioactivity followed by accumulation or screening of the resulting mutants, site-directed introduction of point mutations or knock out of a chromosomally integrated gene encoding for an active enzyme, as described in Sambrook/Fritsch/Maniatis (1989). In addition, the transcriptional repressor FadR may be over expressed to the effect that expression of enzymes involved in the β-oxidation pathway is repressed (Fujita, Y., et al, 2007). The phrase "deletion of a gene", as used herein, means that the nucleic acid sequence encoding said gene is modified such that the expression of active polypeptide encoded by said gene is reduced. For example, the gene may be deleted by removing in-frame a part of the sequence comprising the sequence encoding for the catalytic active centre of the polypeptide. Alternatively, the ribosome binding site may be altered such that the ribosomes no longer translate the corresponding RNA. It would be within the routine skills of the person skilled in the art to measure the activity of enzymes expressed by living cells using standard essays as described in enzymology text books, for example Cornish-Bowden, 1995.

Degradation of fatty acids is accomplished by a sequence of enzymatically catalysed reactions. First of all, fatty acids are taken up and translocated across the cell membrane *via* a transport/acyl-activation mechanism involving at least one outer membrane protein and one inner membrane-associated protein which has fatty acid-CoA ligase activity, referred to in the case of *E. coli* as FadL and FadD / FadK, respectively. Inside the cell, the fatty acid to be degraded is subjected to enzymes catalysing other reactions of the β-oxidation pathway. The first intracellular step involves the conversion of acyl-CoA to enoyl-CoA through acyl-CoA dehydrogenase, the latter referred to as FadE in the case of *E. coli.* The activity of an acyl-CoA dehydrogenase may be assayed as described in the state of art, for example by monitoring the concentration of NADH spectrophotometrically at 340 nm in 100 mM MOPS, pH 7.4, 0.2 mM Enoyl-CoA, 0.4 mM NAD⁺. The resulting enoyl-CoA is converted to 3-ketoacyl-CoA *via* 3-hydroxylacyl-CoA through hydration and oxidation, catalysed by enoyl-CoA hydratase/(R)-3-hydroxyacyl-CoA dehydrogenase, referred to as FadB and FadJ in *E. coli.* Enoyl-CoA hydratase/3-hydroxyacyl-CoA dehydrogenase activity, more specifically formation of the product NADH may be assayed spectrophotometrically as described in the state of the art, for example as outlined for FadE. Finally, 3-ketoacyl-CoA thiolase, FadA and Fadl in *E. coli,* catalyses the cleavage of 3-ketoacyl-CoA, to give acetyl-CoA and the input acyl-CoA shortened by two carbon atoms. The activity of ketoacyl-CoA thiolase may be assayed as described in the state of the art, for example in Antonenkov, V., et al, 1997.

The phrase "a cell having a reduced fatty acid degradation capacity", as used herein, refers to a cell having a reduced capability of taking up and/or degrading fatty acids, particularly those having at least eight carbon chains. The fatty acid degradation capacity of a cell may be reduced in various ways. In particular, the cell according to any aspect of the present invention has, compared to its wild type, a reduced activity of an enzyme involved in the β-oxidation pathway. The term "enzyme involved in the β-oxidation pathway", as used herein, refers to an enzyme that interacts directly with a fatty acid or a derivative thereof formed as part of the degradation of the fatty acid via the β-oxidation pathway. The β-oxidation pathway comprises a sequence of reactions effecting the conversion of a fatty acid to acetyl-CoA and the CoA ester of the shortened fatty acid. The enzyme involved in the β-oxidation pathway may by recognizing the fatty acid or derivative thereof as a substrate, converts it to a metabolite formed as a part of the β-oxidation pathway. For example, the acyl-CoA dehydrogenase (EC 1.3.99.-) is an enzyme involved in the β-oxidation pathway as it interacts with fatty acid-CoA and converts fatty acid-CoA ester to enoyl-CoA, which is a metabolite formed as part of the β-oxidation. In another example, the term "enzyme involved in the β-oxidation pathway", as used herein, comprises any polypeptide from the group comprising acyl-CoA dehydrogenase (EC 1.3.99.-), enoyl-CoA hydratase (EC 4.2.1.17), 3-hydroxyacyl-CoA dehydrogenase EC 1.1.1.35) and 3-keto-acyl-CoA thiolase (EC 2.3.1.16). The acyl-CoA synthetase (EC 6.2.1.1) may catalyse the conversion of a fatty acid to the CoA ester of a fatty acid, *i.e.* a molecule, wherein the functional group -OH of the carboxy group is replaced with -S-CoA and introducing the fatty acid into the β-oxidation pathway. For example, the polypeptides FadD and FadK in *E. coli* (accession number: BAA15609.1 and NP_416216.4, respectively) are acyl-CoA dehydrogenases. In one example, the term "acyl-CoA dehydrogenase", as used herein, may be a polypeptide capable of catalysing the conversion of an acyl-CoA to enoyl-CoA, as part of the β-oxidation pathway. For example, the polypeptide FadE in *E. coli* (accession number: BAA77891.2) may be an acyl-CoA dehydrogenase. The term "enoyl-CoA hydratase", as used herein, also referred to as 3-hydroxyacyl-CoA dehydrogenase, refers to a polypeptide capable of catalysing the conversion of enoyl-CoA to 3-ketoacyl-CoA through hydration and oxidation, as part of the β-oxidation pathway. For example, the polypeptides FadB and FadJ in *E. coli* (accession numbers: BAE77457.1 and P77399.1, respectively) are enoyl-CoA hydratases. The term "ketoacyl-CoA thiolase", as used herein, may refer to a polypeptide capable of catalysing the cleaving of 3-ketoacyl-CoA, resulting in an acyl-CoA shortened by two carbon atoms and acetyl-CoA, as the final step of the β-oxidation pathway. For example, the polypeptides FadA and Fadl in *E. coli* (accession number: YP_491599.1and P76503.1, respectively) are ketoacyl-CoA thiolases.

According to any aspect of the present invention, the cell may comprise a further genetic mutation that increases the expression relative to the wild type cell of an enzyme E₁₀, an alkane oxidase capable of catalysing the oxidation of butylacetate to 4-acetyl-butyric acid and/or ester thereof.

Various alkane oxidases have been described in the literature, for example basidiomycete hemethiolate peroxidases (Gutierrez, A., 2011, Lo Piccolo, L., 2011, Grant, C., 2011, Koch, D., 2009). In one example, the alkane oxidase may be an alkB type alkane oxidase. AlkB is an oxidoreductase from the *Pseudomonas putida* AlkBGT system, dependent on two auxiliary polypeptides, AlkG and AlkT. AlkT is a FAD-dependent rubredoxin reductase transferring electrons from NADH to AlkG. AlkG is a rubredoxin, an iron-containing redox protein functioning as a direct electron donor to AlkB. In one example, the term "alkB type alkane oxidase", as used herein, refers to AlkB from *Pseudomonas putida* Gpo1 (Access code: CAB54050.1, any access code used in the application refers to the respective sequence from the Genbank database run by the NCBI, wherein the release referred to is the one online on the 6^{th} May, 2015) or a variant thereof.

In particular, the enzyme E₁₀ may be selected from the group consisting of rubredoxin-dependent alkane oxidase (EC 1.14.15.3), cytochrome P450 enzyme (EC 1.14.x.x), xylene monooxygenase (EC:1.14.13.-; EC:1.18.1.3) and methane monoxygenase (EC 1.14.13.25; EC 1.14.18.3).

The term "rubredoxin-dependent alkane oxidase", as used herein refers to an oxidoreductase that recognises as its substrate an alkane receiving electrons *via* a rubredoxin, the latter being, an iron-sulphur protein having an α+β class fold with 2 α helices and 2 to 3 β-strands transferring electrons to the alkane oxidase and AlkG from *Pseudomonas putida* or a variant thereof. Examples include AlkG from *Pseudomonas putida.* The term "cytochrome P450 enzyme", as used herein, refers to an oxidoreductase having a P450-type cytochrome having, in its CO-bound form an absorption band at 450 nm and may be capable of oxidizing an alkane. Examples include cytochrome P450 BM-3 from *Bacillus megaterium* (Koch, D. J., 2009.) The term "xylene monooxygenase", as used herein, refers to a membrane-spanning, non-heme diiron enzyme oxidoreductase with a histidine-rich active site and preferably capable of oxidizing an alkane. Examples include XylM from *Pseudomonas putida* (Austin, R. N., 2003). The term "methane monooxygenase", as used herein, refers to an oxidoreductase that is either a soluble methane monooxygenase comprising di-iron center bridged by an oxygen atom (Fe-O-Fe) and comprising three protein components, a hydroxylase, a β unit, and a reductase, from a methanotropic bacterium or is a particulate methane monooxygenase, a membrane-protein in a methanotrophic bacterium, comprising a copper-containing active site. Examples of soluble and particulate methane monooxygenases comprises the soluble methane monooxygenase from *Methylosinus trichosporium* OB3b (A C Rosenzweig, 1993) and the methane monooxygenase from *Methylococcus capsulatus* (Bath) (Nguyen, H. H. T., 1998).

The cell according to any aspect of the present invention may also have increased expression relative to a wild type cell of at least one enzyme that may allow the cell according to any aspect of the present invention to convert carbon sources containing CO and/or CO₂ to butanol and/or acetic acid. In particular, the cell may comprise enzymes that are able to catalyse the acetyl-CoA or Wood-Ljungdahl pathway and the ethanol-carboxylate fermentation pathway to produce butanol and/or acetic acid. The butanol and/or acetic acid may then be esterified by state of the art methods like acid catalysts and/or lipases as known in the art to produce butylacetate.

The phrase "wild type" as used herein in conjunction with a cell or microorganism may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

A skilled person would be able to use any method known in the art to genetically modify a cell or microorganism. According to any aspect of the present invention, the genetically modified cell may be genetically modified so that in a defined time interval, within 2 hours, in particular within 8 hours or 24 hours, it forms at least twice, especially at least 10 times, at least 100 times, at least 1000 times or at least 10000 times more carboxylic acid and/or the respective carboxylic acid ester than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (carboxylic acid) in the nutrient medium.

In another aspect of the present invention, there is provided a method of producing acetylhomoserine from at least one carbon source, the method comprising a step of contacting an enzyme E₁ with 4-acetyl-butyric acid and/or ester thereof, wherein the enzyme E₁ is capable of catalysing the hydroxylation of 4-acetyl-butyric acid and/or ester thereof to an alpha-hydroxy acetyl butanone. In particular, the enzyme E₁ may be cytochrome P450 BM3 (CYP102A1). This step may be a step in any biotechnological method of producing acetylhomoserine, where 4-acetyl-butyric acid and/or ester thereof may be found as a substrate and/or raw material to be hydroxylated.

According to another aspect of the present invention, there is provided a method of producing acetylhomoserine from 4-acetyl-butyric acid and/or ester thereof, the method comprising the step of contacting the cell according to any aspect of the present invention with a medium comprising 4-acetyl-butyric acid and/or ester thereof.

The term "contacting", as used herein, means bringing about direct contact between the of 4-acetyl-butyric acid and/or ester thereof with the enzyme E₁ and/or cell according to any aspect of the present invention in an aqueous solution. For example, the cell and 4-acetyl-butyric acid and/or ester thereof may be in the same container allowing the enzyme to work on the 4-acetyl-butyric acid and/or ester thereof. If the 4-acetyl-butyric acid and/or ester thereof is soluble and may be taken up by the cell or can diffuse across biological membranes, it may simply be added to the cell according to any aspect of the present invention in an aqueous solution. In case it is insufficiently soluble, it may be solved in a suitable organic solvent prior to addition to the aqueous solution. The person skilled in the art is able to prepare aqueous solutions of 4-acetyl-butyric acid and/or ester thereof having insufficient solubility by adding suitable organic and/or polar solvents. Such solvents may be provided in the form of an organic phase comprising liquid organic solvent. In one example, the organic solvent or phase may be considered liquid when liquid at 25 °C and standard atmospheric pressure.

The term "an aqueous solution" or "medium" comprises any solution comprising water, mainly water as solvent that may be used to keep the cell according to any aspect of the present invention, at least temporarily, in a metabolically active and/or viable state and comprises, if such is necessary, any additional substrates. The person skilled in the art is familiar with the preparation of numerous aqueous solutions, usually referred to as media that may be used to keep inventive cells, for example LB medium in the case of *E. coli.* It is advantageous to use as an aqueous solution a minimal medium, i.e. a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium.

According to a further aspect of the present invention, there is provided a method comprising a step of contacting the cell according to any aspect of the present invention with a medium comprising butylacetate.

According to another aspect of the present invention, there is provided a method of producing acetylhomoserine from a carbon source, the method comprising the steps of:
(i) contacting a reaction mixture comprising a first and a second microorganism with the carbon source, wherein
   - the first microorganism is an acetogenic microorganism capable of converting the carbon source to acetate and/or ethanol;
   - the second microorganism is capable of carrying out the ethanol carboxylate fermentation pathway and convert the acetate and/or ethanol to form butyric acid; and
   - the first microorganism is further capable of converting the butyric acid to butanol;
(ii) esterifying the butanol to form butylacetate;
(iii) contacting the butylacetate with at least one enzyme E₃, an alkane oxidase that is capable of oxidising butylacetate to form 4-acetyl butyric acid and/or ester thereof; and
(iv) contacting the 4-acetyl butyric acid and/or ester thereof with a cell according to any aspect of the present invention.

In one example, the first and second microorganisms are added simultaneously. In another example, the first and second microorganisms are added consequently. The concentration of the first and second microorganisms may be constantly maintained to keep the reaction progressing. In one example, before the second microorganism is added, the concentration of ethanol and/or acetate is measured to ensure an optimum concentration is reached for the second microorganism. In particular, the acetate and/or ethanol concentration may be at an optimum level for there to be sufficient substrate in the aqueous medium for the second microorganism to form at least butyric acid. A skilled person would be able to easily determine the suitable time to include the second organism into the aqueous medium.

In another example, the first and second microorganisms are added simultaneously. In this example, the second microorganism may not be active (i.e. may remain latent) until a sufficient amount of acetate and/or ethanol is made available by the activity of the first microorganism. The presence of either microorganism in the aqueous medium does not disrupt or hinder the activity and/or efficiency of the other.

An advantage of the present invention may be that much more favorable CO₂/CO mixtures of raw materials can be used. These various sources include natural gas, biogas, coal, oil, plant residues and the like. Another advantage of the method may be the high carbon yield. This is made possible by the return of formed CO₂. Namely, the CO₂ can be reacted in the first stage back to acetic acid. Another advantage may lie in greater flexibility with regard to the fermentation conditions used, as any acetogenic and any microorganism capable of carrying out the ethanol-carboxylate fermentation pathway may be used in combination for the actual production of higher alcohols. Another advantage of the present invention may be that since the second microorganism may function and/or produce an acid from the acetate and/or ethanol in the presence of CO, both the first and second microorganisms may be present in a homogenous mixture for the production of higher alcohols from a carbon source comprising CO. This feature of the second microorganism enables the production of higher alcohols from a carbon source like CO to be a one step process making the process more efficient and the yield greater. Surprisingly, because of this advantage of the second microorganism, the one-step procedure for making higher alcohols may be carried out in a single fermenter without an intermediate separation step. There may also be an increased concentration of the final product using this one step procedure. This is surprising as Baffert C., 2011 and Thauer, R.K., 1973 both teach that hydrogenases were inhibited in the presence of CO.

The term "acetogenic bacteria" as used herein refers to a microorganism which is able to perform the Wood-Ljungdahl pathway and thus is able to convert CO, CO₂ and/or hydrogen to acetate. These microorganisms include microorganisms which in their wild-type form do not have a Wood-Ljungdahl pathway, but have acquired this trait as a result of genetic modification. Such microorganisms include but are not limited to *E. coli* cells. These microorganisms may be also known as carboxydotrophic bacteria. Currently, 21 different genera of the acetogenic bacteria are known in the art (Drake et al., 2006), and these may also include some *clostridia* (Drake & Kusel, 2005). These bacteria are able to use carbon dioxide or carbon monoxide as a carbon source with hydrogen as an energy source (Wood, 1991). Further, alcohols, aldehydes, carboxylic acids as well as numerous hexoses may also be used as a carbon source (Drake et al., 2004). The reductive pathway that leads to the formation of acetate is referred to as acetyl-CoA or Wood-Ljungdahl pathway.

In particular, the acetogenic bacteria may be selected from the group consisting of *Acetoanaerobium notera (ATCC* 35199), *Acetonema longum (DSM 6540), Acetobacterium carbinolicum (DSM* 2925), *Acetobacterium malicum (DSM* 4132), *Acetobacterium species no. 446 (*Morinaga et al., 1990, J. Biotechnol., Vol. 14, p. 187-194*),Acetobacterium wieringae (DSM 1911), Acetobacterium woodii (DSM 1030), Alkalibaculum bacchi (DSM 22112), Archaeoglobus fulgidus (DSM 4304), Blautia producta (DSM 2950, formerly Ruminococcus productus, formerly Peptostreptococcus productus), Butyribacterium methylotrophicum (DSM* 3468), *Clostridium aceticum (DSM* 1496), *Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium Ijungdahlii (DSM 13528), Clostridium Ijungdahlii C-01 (ATCC 55988), Clostridium Ijungdahlii ERI-2 (ATCC 55380), Clostridium Ijungdahlii O-52 (ATCC 55989), Clostridium mayombei (DSM* 6539), *Clostridium methoxybenzovorans (DSM 12182), Clostridium ragsdalei (DSM 15248), Clostridium scatologenes (DSM 757), Clostridium species ATCC 29797 (*Schmidt et al., 1986, Chem. Eng. Commun., Vol. 45, p. 61-73), *Desulfotomaculum kuznetsovii (DSM 6115), Desulfotomaculum thermobezoicum subsp. thermosyntrophicum (DSM 14055), Eubacterium limosum (DSM 20543), Methanosarcina acetivorans C2A (DSM* 2834), *Moorella sp. HUC22-1 (*Sakai et al., 2004, Biotechnol. Let., Vol. 29, p. 1607-1612*), Moorella thermoacetica (DSM 521, formerly Clostridium thermoaceticum), Moorella thermoautotrophica (DSM 1974), Oxobacter pfennigii (DSM 322), Sporomusa aerivorans (DSM* 13326), *Sporomusa ovata (DSM 2662), Sporomusa silvacetica (DSM 10669), Sporomusa sphaeroides (DSM 2875), Sporomusa termitida (DSM 4440) and Thermoanaerobacter kivui (DSM 2030, formerly Acetogenium kivui).* More in particular, the strain ATCC BAA-624 of *Clostridium carboxidivorans* may be used. Even more in particular, the bacterial strain labelled "P7" and "P11" of *Clostridium carboxidivorans* as described for example in U.S. 2007/0275447 and U.S. 2008/0057554 may be used.

Another particularly suitable bacterium may be *Clostridium Ijungdahlii.* In particular, strains selected from the group consisting of *Clostridium Ijungdahlii* PETC, *Clostridium Ijungdahlii* ERI2, *Clostridium Ijungdahlii* COL and *Clostridium Ijungdahlii* O-52 may be used in the conversion of synthesis gas to hexanoic acid. These strains for example are described in WO 98/00558, WO 00/68407, ATCC 49587, ATCC 55988 and ATCC 55989.

The acetogenic bacteria may be used in conjunction with a second microorganism that may be capable of carrying out the ethanol-carboxylate fermentation pathway. In one example, both an acetogenic bacteria and a second microorganism that may be capable of carrying out the ethanol-carboxylate fermentation pathway may be used to produce a butyric acid from the carbon source. The butyric acid may then be converted to butanol. In one example, a single microorganism may be able to carry out the activity of an acetogenic bacterium and the ethanol-carboxylate fermentation pathway. For example, the bacterium may be *C.Carboxidivorans.*

In one example, the ethanol and/or acetate may be converted to butyric acid in the presence of the second microorganism capable of carrying out the ethanol-carboxylate fermentation pathway. The ethanol-carboxylate fermentation pathway is described in detail at least in Seedorf, H., et al., 2008. In particular, the second organism may be selected from the group consisting of *Clostridium kluyveri, C.Carboxidivorans* and the like. These second microorganisms include microorganisms which in their wild-type form do not have an ethanol-carboxylate fermentation pathway, but have acquired this trait as a result of genetic modification. In particular, the second microorganism may be *Clostridium kluyveri.*

In another example, the second microorganism may be a wild type organism that expresses at least one enzyme selected from the group consisting of E₁₁ to E₂₁, wherein E₁₁ is an alcohol dehydrogenase (adh), E₁₂ is an acetaldehyde dehydrogenase (ald), E₁₃ is an acetoacetyl- CoA thiolase (thl), E₁₄ is a 3-hydroxybutyryl-CoA dehydrogenase (hbd), E₁₅ is a 3-hydroxybutyryl-CoA dehydratase (crt), E₁₆ is a butyryl-CoA dehydrogenase (bcd), E₁₇ is an electron transfer flavoprotein subunit (etf), E₁₈ is a coenzyme A transferase (cat), E₁₉ is an acetate kinase (ack), E₂₀ is phosphotransacetylase (pta) and E₂₁ is a transhydrogenase. In particular, the wild type second microorganism according to any aspect of the present invention may express at least E₁₂, E₁₃ and E₁₄. Even more in particular, the wild type second microorganism according to any aspect of the present invention may express at least E₁₄.

In another example, the second microorganism according to any aspect of the present invention may be a genetically modified organism that has increased expression relative to the wild type microorganism of at least one enzyme selected E₁₁ to E₂₁ wherein E₁₁ is an alcohol dehydrogenase (adh), E₁₂ is an acetaldehyde dehydrogenase (ald), E₁₃ is an acetoacetyl-CoA thiolase (thl), E₁₄ is a 3-hydroxybutyryl-CoA dehydrogenase (hbd), E₁₅ is a 3-hydroxybutyryl-CoA dehydratase (crt), E₁₆ is a butyryl-CoA dehydrogenase (bcd), E₁₇ is an electron transfer flavoprotein subunit (etf), E₁₈ is a coenzyme A transferase (cat), E₁₉ is an acetate kinase (ack) E₂₀ is phosphotransacetylase (pta) and E₂₁ is a transhydrogenase. In particular, the genetically modified second microorganism according to any aspect of the present invention may express at least enzymes E₁₂, E₁₃ and E₁₄. Even more in particular, the genetically modified second microorganism according to any aspect of the present invention may express at least E₁₄. The enzymes E₁₁ to E₂₁ may be isolated from *Clostridium kluyveri.* A skilled person may be capable of measuring the activity of each of these enzymes using methods known in the art. In particular, the activity of enzymes E₁₁ and E₁₂ may be measured using the assays taught at least in Hillmer P., 1972, Lurz R., 1979; the activity of enzyme E₁₂ may also be measured using the assay taught in Smith L.T., 1980; the activity of enzymes E₁₃ and E₁₄ may be measured using the assays taught at least in Sliwkowski M.X., 1984; the activity of E₁₄ may also be measured using the assay taught in Madan, V.K., 1972; the activity of E₁₅ may also be measured using the assay taught in Bartsch, R.G., 1961; the activity of enzymes E₁₆ and E₁₇ may be measured using the assay taught in Li, F., 2008; the activity of E₁₇ may also be measured using the assay taught in Chowdhury, 2013; the activity of E₁₈ may be measured using the assay taught in Stadman, 1953; the activity of E₁₉ may be measured using the assay taught in Winzer, K., 1997; the activity of E₂₀ may be measured using the assay taught in Smith L.T., 1976; and the activity of E₂₁ may be measured using the assay taught in Wang S, 2010.

According to any aspect of the present invention, the first and/or second microorganism may be a genetically modified microorganism. The genetically modified cell or microorganism may be genetically different from the wild type cell or microorganism. The genetic difference between the genetically modified microorganism according to any aspect of the present invention and the wild type microorganism may be in the presence of a complete gene, amino acid, nucleotide etc. in the genetically modified microorganism that may be absent in the wild type microorganism. In one example, the genetically modified microorganism according to any aspect of the present invention may comprise enzymes that enable the microorganism to produce at least one carboxylic acid. The wild type microorganism relative to the genetically modified microorganism according to any aspect of the present invention may have none or no detectable activity of the enzymes that enable the genetically modified microorganism to produce at least one carboxylic acid. As used herein, the term 'genetically modified microorganism' may be used interchangeably with the term 'genetically modified cell'. The genetic modification according to any aspect of the present invention may be carried out on the cell of the microorganism.

Even more in particular, according to any aspect of the present invention, the acid is produced in the presence of at least one acetogenic microorganism and *Clostridium kluyveri.* In one example, the acetogenic microorganism may be *Clostridium Ijungdahlii* or *Clostridium ragsdahlei.*

The term "acetate" as used herein, refers to both acetic acid and salts thereof, which results inevitably, because as known in the art, since the microorganisms work in an aqueous environment, and there is always a balance between salt and acid present. According to any aspect of the present invention, the carbon source may be synthesis gas. Synthesis gas can for example be produced as a by-product of coal gasification. Accordingly, the microorganism of the mixed culture according to any aspect of the present invention may be capable of converting a substance which is a waste product into a valuable resource. In another example, synthesis gas may be a by-product of gasification of widely available, low-cost agricultural raw materials for use with the mixed culture of the present invention to produce at least one higher alcohol.

There are numerous examples of raw materials that can be converted into synthesis gas, as almost all forms of vegetation can be used for this purpose. In particular, raw materials are selected from the group consisting of perennial grasses such as miscanthus, corn residues, processing waste such as sawdust and the like.

In general, synthesis gas may be obtained in a gasification apparatus of dried biomass, mainly through pyrolysis, partial oxidation and steam reforming, wherein the primary products of the synthesis gas are CO, H₂ and CO₂.

Usually, a portion of the synthesis gas obtained from the gasification process is first processed in order to optimize product yields, and to avoid formation of tar. Cracking of the undesired tar and CO in the synthesis gas may be carried out using lime and/or dolomite. These processes are described in detail in for example, Reed, 1981.

Mixtures of sources can be used as a carbon source.

A skilled person would understand the other conditions necessary to carry out the method according to any aspect of the present invention. In particular, the conditions in the container (e.g. fermenter) may be varied depending on the first and second microorganisms used. The varying of the conditions to be suitable for the optimal functioning of the microorganisms is within the knowledge of a skilled person.

In one example, the method according to any aspect of the present invention may be carried out in an aqueous medium with a pH between 5 and 8, 5.5 and 7. The pressure may be between 1 and 10 bar.

In particular, the aqueous medium may comprise a carbon source comprising CO and/or CO₂. More in particular, the carbon source comprising CO and/or CO₂ is provided to the aqueous medium in a continuous gas flow. Even more in particular, the continuous gas flow comprises synthesis gas. In one example, the gases are part of the same flow/stream. In another example, each gas is a separate flow/stream provided to the aqueous medium. These gases may be divided for example using separate nozzles that open up into the aqueous medium, frits, membranes within the pipe supplying the gas into the aqueous medium and the like.

In particular, the reaction mixture according to any aspect of the present invention (i.e. mixture of the first microorganism- the acetogenic organism, the second microorganism, and the carbon source can be employed in any known bioreactor or fermenter to carry out any aspect of the present invention.

Acetylhomoserine is one of the substrates used in the production of methionine. In particular, the reaction is:
O-acetyl-L-homoserine + methanethiol → L-methionine + acetate

Methanethiol can be chemically synthesised from H₂S and MeOH. O-acetyl-L-homoserine may be produced according to any aspect of the present invention. By combining homoserine with methanethiol, L-methionine may then be produced.

### BRIEF DESCRIPTION OF FIGURES

The inventions are further illustrated by the following figures and non-limiting examples from which further embodiments, aspects and advantages of the present invention may be taken.

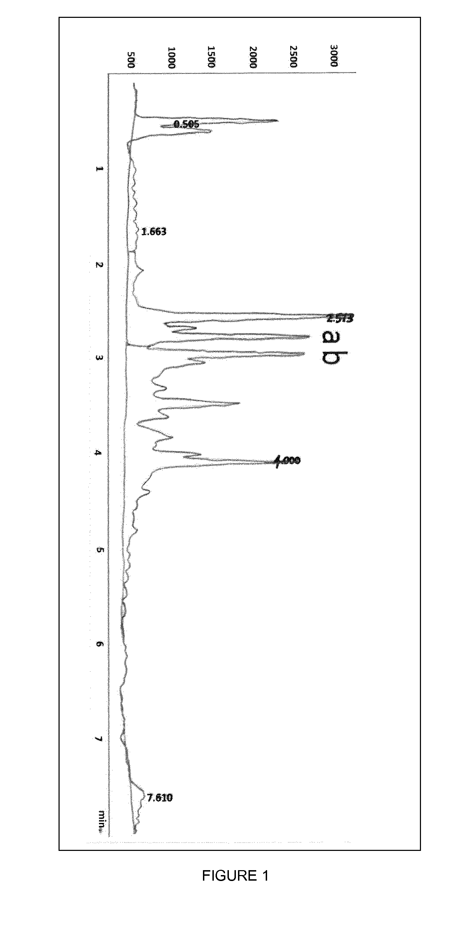

Figure 1 is LC-MS scan for mass 190.08+1 after conversion of EAB with P450 BM3 variant M2. a = product peak 1 with mass 191. b = product peak 2 with mass 191.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### Conversion of ethyl 4-acetoxybutanoate with P450 BM3

Ethyl acetoxy-butanoate (EAB) was converted with P450 BM3 variant M2 employing O₂ as oxidant.

### General methodology

The 3.1 kb P450 BM3 gene from *Bacillus megaterium* (CYP102A1) was cloned into the 2 kb pET22b-derived pALXtreme-1 a plasmid (Blanusa et al., 2011). For expression of the P450 BM3 monooxygenase (EC 1.14.14.1) the pALXtreme-1, a plasmid, was transformed into chemical competent *E. coli* BL21 Gold (DE3)-derived E. coli laql^{Q1} Gold (DE3) cells. Preparation of chemical competent cells was achieved following a standard protocol generating 108 clones per µξ pUC19 (Inoue et al., 1990). All plasmid preparations were performed using the QIAGEN QIAprep Spin Miniprep Kit (Hilden, Germany). All chemicals used were of analytical grade and purchased from Sigma Aldrich (Steinheim, Germany), abcr (Karlsruhe, Germany) or AppliChem (Darmstadt, Germany). Oligonucleotides used for mutagenesis were obtained from Eurofins MWG Operon (Ebersberg, Germany) and produced at HPLC purity. Enzymes were received from New England Biolabs (Frankfurt, Germany) and Sigma Aldrich (Steinheim, Germany). dNTPs were purchased from Fermentas (St. Leon-Rot, Germany).

For protein activity measurement of the P450 BM3 mutant libraries, frozen pellets of grown expression cultures stored for 4h at -20° C in 96-deep-well plates were resuspended by retro-pipetting in 150 µl 50 mM potassium phosphate buffer (KPi) pH 7.5. Additional 150 µl 50 mM KPi pH 7.5 was added containing 5 mg/ml lysozyme. Plates were incubated for 1 h at 900 rpm and 37°C in an Infors HT Microtron shaker to ensure entire cell lysis. Plates with lysed cells were centrifuged for 15 min at 3200 g and 4° C in an Eppendorf 581 OR centrifuge to spin down cell debris. The clear supernatant was used for assaying monooxygenase activity by measuring NADPH depletion at 340 nm in a Tecan Sunrise MTP reader (Groeding, Austria; Glieder & Meinhld, 2003). 96-well quarz glass MTPs (Hellma, Muellheim, Germany) were used during all measurements with p-xylene since the substrate reacts with most poly-propylene and poly-styrene based materials. A reaction mixture in MTP format contained 1.5 % DMSO, 200 mM p-xylene, 10 to 50 µl cell lysate and 0.16 mM NADPH in a final volume of 250 µl 50 mM KPi buffer pH 7.5. The amount of lysate had to be adjusted in a way that linear depletion of NADPH could be monitored reliably for 2 min. The reaction was stopped by pipetting 25 µl quenching buffer (4 M urea in 0.1 M NaOH) in the reaction mixture, followed by addition of 20 µl 4-aminoantipyrine (4-AAP) (5 mg/ml) and 20 µl potassium peroxodisulphate (5 mg/ml) for phenolic product quantification (Wong et al., 2005). After 30 min incubation at 750 rpm on an Eppendorf MixMate MTP shaker (Hamburg, Germany) the amount of 2.5-DMP was quantified at 509 nm absorbance in a Tecan Sunrise MTP reader (Wong et al.,supra). Mutants for next rounds of saturation were selected based upon best performance for NADPH depletion as well as highest formation of 2,5-DMP. Improved variants of P450 BM3 were sequenced at MWG Eurofins DNA (Ebersberg, Germany) and analyzed using Clone Manager 9 Professional Edition software (Scientific & Educational Software, Cary, NC, USA).

### Generation of P450 BM3 mutants

Three amino acid residues were chosen for iterative NNK saturation (R47, Y51, and I401). Amino acid residues R47 and Y51 were simultaneously saturated meanwhile site I401 was saturated as a single position. Oligonucleotides were designed for optimal PCR product yields according to recommendations of user's manual from Stratagene's QuikChange Site-directed mutagenesis Kit (Stratagene, La Jolla, CA, USA). A standard PCR set-up contained 20 ng plasmid DNA, 5 U Phusion DNA polymerase, 1 x Phusion DNA polymerase buffer, 0.2 mM dNTP mix and 0.4 µM of forward and reverse primer (Table 4). All PCRs were performed in thin wall PCR tubes (Saarstedt, Nuembrecht, Germany) and using an Eppendorf Mastercycler proS (Hamburg, Germany). The cycling protocol was performed according to Stratagene's QuikChange Site-directed mutagenesis Kit manual instruction (Stratagene, La Jolla, CA, USA). Temperatures and incubation times during each PCR cycle were adjusted to provider's recommendation for Phusion DNA polymerase (New England Biolabs, Frankfurt, Germany). Annealing temperature during each PCR cycle was adjusted to 55 °C for 30 s. Successful PCR amplifications were verified on 0.8% agarose gel according to a standard protocol (Maniatis et al. 1982). Digestion of template DNA was achieved by addition of 20 U Dpnl to 50 µl PCR mixture followed by incubation at 37°C for 3 h. The PCR products were transformed without further purification into chemically competent *E .coli* Gold (DE3) laql^{Q1} cells using a heat shock transformation protocol (Hanahan *et al.,* 1991). Recovered cells were plated and incubated overnight at 37° C on LB agar plates containing 100 Mg/ml kanamycin. Obtained colonies were transferred with sterile toothpicks into Greiner BioOne 96-well microtiterplates (MTP) (Frickenhausen, Germany) pre-filled with 100 µl LB media and 100 Mg/ml kanamycin. The plates were incubated for 14 h at 900 rpm and 37° C in an Infors HT Microtron shaker (Bottmingen, Switzerland) followed by addition of 100 µl of 50% glycerol (W/V). All MTPs were stored at -80° C prior to expression of mutant libraries.

**Table 4. Oligonucleotides used for NNK saturation of targeted amino acid residues.**

| Position | Primer name | SEQ ID No: | Primer sequence 5' - 3' |
|---|---|---|---|
| R47/Y51 | R47/Y51 Fw | 6 | CGAGGCGCCTGGTNNKGTAACGCGCNNKTTATCAAGTCAGCG |
| | | 7 | CGCTGACTTGATAAMNNGCGCGTTACMNNACCAGGCGCCTCG |
| | R47/Y51 Rv | | |
| 1401 | 1401 Fw | 8 | CAGCGTGCGTGTNNKGGTCAGCAGTTC |
| | 1401 Rw | 9 | GAACTGCTGACCMNNACACGCACGCTG |

### Expression and purification of P450 BM3 mutants

The grown culture of the P450 BM3 mutant were centrifuged for 15 min at 2900 g and 4° C in an Eppendorf 5804R centrifuge (Hamburg, Germany) for removal of residual expression media. Obtained pellets were washed in 50 mM KPi pH 7.8 and spin down again applying the same centrifugation program (15 min; 2900 g; 4° C). The supernatant was removed and pellets were stored at -20° C for 12 h. For purification, frozen cell pellets were resuspended in 10 ml 50mM KPi buffer pH 7.8 prior to disruption in an Avestin EmulsiFlex-C3 high-pressure homogenizer (Ottawa, ON, Canada) by applying three cycles of 1500 bar pressure. Cell debris was removed by centrifuging homogenized samples for 30 min at 16000 g and 4° C in a Sorvall RC-6 Plus ultracentrifuge (Thermo Scientific, Rockford, IL, USA). Removal of small insoluble particles was achieved by pressing the supernatant through a 0.45 uM membrane filter. The filtered cell lysate was loaded on a Kronlab TAC15/ 125PE5-AB-2 column (YMC Europe GmbH, Dinslaken, Germany) prefilled with Toyopearl DEAE 650S anion exchange matrix (Tosoh Bioscience, Stuttgart, Germany). An AKTAprime Plus chromatography system with UV- detection (GE Healthcare, Muenchen, Germany) was used for purification and collection of eluted protein samples. A standard protocol was applied to purify P450 BM3 wild-type as well as the variant M2 (R47S/Y51W/I401M) (Schwaneberg et al.1999). Eluted protein samples were collected and purity was estimated on a 10% sodium dodecyl sulfate-polyacrylamide gel electrophoresis (SDS-PAGE). Fractions containing active and approximately 90% pure protein, were pooled in an Amicon Ultra-4 centrifugation tube (30 kDa cutoff; Millipore, Schwalbach, Germany) and concentrated to a final volume of 2 ml. Concentrated protein samples were desalted and equilibrated in 50 mM KPi pH 7.5 buffer using a PD-10 gel-filtration column (GE Healthcare, Muenchen, Germany). Finally enzyme samples were shock-frozen for long time storage in liquid N2 and lyophilized in an Alpha 1 -2 LD plus freeze-dryer (Christ, Osterode am Harz, Germany) for 48 h at -54° C.

The essential NADPH cofactor was regenerated using a glucose dehydrogenase (GDH) and glucose as shown in Scheme 1.

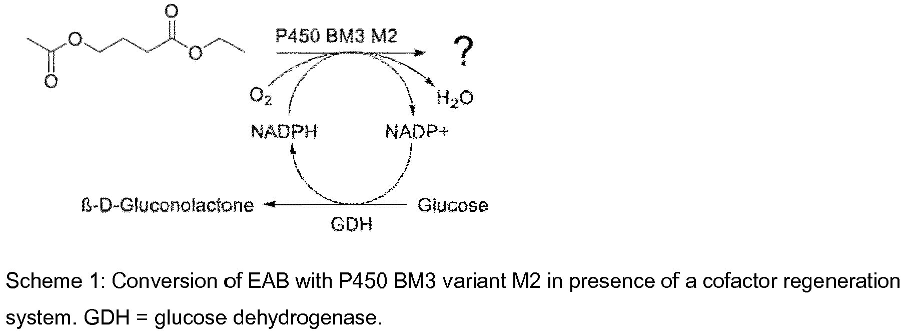

### Reactions were done in 1 mL scale (KPi-buffer, pH 7.5, 0.1 M) containing following components:

12 U GDH
100 mM glucose
400 µM NADP+
50 mM EBA (substrate) in DMSO (5 % V/V DMSO final)
50 mg cell free freeze dried lysate of P450 BM3 variant M2 (SEQ ID NO:2)

### Control reactions:

a) Empty vector; 50 mg of freeze dried *E. coli* BL21 (DE3) cells containing no P450 enzyme
b) Reaction containing no substrate (5 % V/V DMSO supplemented instead)

### GC-MS analysis

The product was recovered by liquid-liquid extraction using 500 µL EtOAc as organic phase. No product could be identified by GC-MS.

### LC-MS analysis

LC-MS analysis as formation of unstable (polar) intermediates/products occured (e.g. emiacetals), depending on the regioselectivity of the P450 monooxygenase catalyst (Scheme 2).

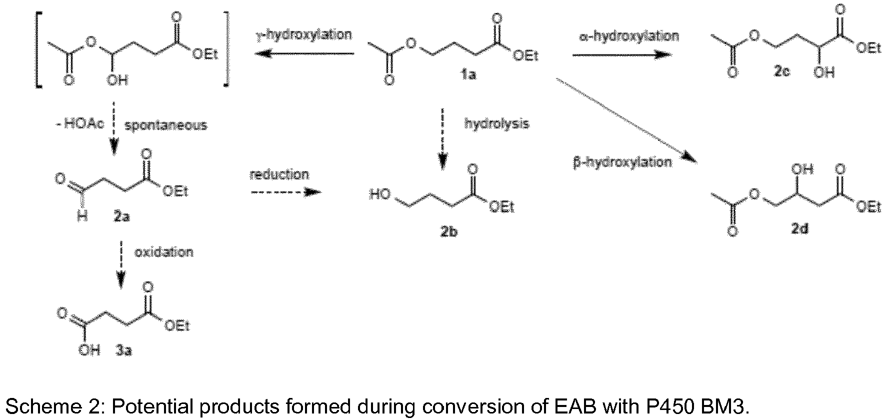

For analysis, 100 µl of the reaction volume were mixed with 100 µl acetonitrile followed by centrifugation to remove insoluble particles. The supernatant was diluted 1:50 in H2O/acetonitrile (50:50; V/V) followed by injection into the LC-MS.
To identify potentially formed side-products, LC-MS scans were done for following masses:
a) 130.06+1 = Product 2a
b) 146.06+1 = Product 3a
c) 132.08+1 = Product 2b
d) 190.08+1 = Product 2c and 2d
e) 174.09+1 = Substrate (EAB) 1a

### Mass-scanning of reaction samples

A mass-scanning was done to identify potential products formed during conversion of EAB with P450 BM3. At 2.96 min retention time a peak was detected that elutes before the substrate (retention time 3.699) indicating formation of a more polar compound. The same product peak was not detected in the control reaction which contains *E. coli* cells without P450 BM3.

### Analysis of reaction samples for mass 190.08+1 (target product 2c and 2d)

Detection of mass 190.08+1 revealed formation of two product peaks at 2.9 and 3.0 min that were only present in the reaction samples containing P450 BM3 variant M2 and EAB as substrate (Figure 1) Both peaks were not detectable in the control reactions nor in the substrate as possible contamination. In addition, the product peaks elute earlier than the substrate (3.699 min) indicating a higher polarity possibly due to a hydroxylation of EAB.

### Analysis of reaction samples for mass 132.08+1 (Product 2b) - potential hydrolysis of the acetoxygroup

A large peak signal was obtained for mass 132.06+1 (-20 % area) most likely corresponding to the formation of compound 2b (Scheme 2).

Formation of an unstable hemiacetal by P450 BM3 variant M2 is very unlikely since the same compound was also detected in the EV control reaction and no aldehyde formation occurred (2a, Scheme 1). Accordingly, intrinsic *E. coli* hydrolases were most likely responsible for the catalysing the formation of 2b.

P450 BM3 variant M2 possibly catalyses the formation of target 2c and unwanted side product 2d in rather equal amount. was hydrolyzed by *E. coli* endogeneous enzymes (e.g. esterases, lipases).

### NMR Analysis

NMR was also carried out on the reaction mixtures. The results also showed that 450 BM3 variant M2 possibly catalyses the formation of target 2c and unwanted side product 2d.

### REFERENCES

A C Rosenzweig, (1993) Nature, 366, 537-543
Figge RM (2006), ed Wendisch VF, Microbiol Monogr (5) Amino acid biosynthesis p 164- 185
Gutierrez, A., (2011), Arch. Biochem. Biophys. 514 (1-2), 33-43,
Lo Piccolo, L., (2011), Appl.. Environm. Microbiol. 77 (4), 1204-12013
Grant, C., (2011) Enzyme and Microbial Technology, 480-486) and
Koch, D. J., (2009) Appl. and Env. Microbiology, 337-344
Koch, D. J., (2009) Appl. And Environm. Microbiol. 75(2), 337-344).
Austin, R. N., (2003) J. Biol. Inorg. Chem. 8, 733-740.
Nguyen, H. H. T., (1998), J. Biol. Chem. 273, 7957-7966
J Sangster, Octanol-Water Partition Coefficients: Fundamentals and Physical Chemistry, Vol. 2 of Wiley Series in Solution Chemistry, John Wiley & Sons, Chichester, 1997
Girhard, M., et al., (2007) Biochem. Biophys. Res. Commun., 362, 114-119
Adam, W. et al., (1998) J. Am. Chem. Soc., 120, 11044
Adam, W. et al., (1996) Tetrahedron: Asymmetry, 7, 2287
H. Schütte, et al., (1984), Appl. Microbiol. Technol., 19, 167-176
W. Hummel, et al., (1985), Appl. Microbiol. Technol. 1985, 21, 7-15
Malca S.H., et al (2011) Biochimica et Biophysica Acta 1814 257-264
Getrey et. al., (2014) Green Chem., 16:1104
Abrahamson, M.J., Angew. Chem. Int. Ed. 2012, 51, 3969 -3972
Matsunaga M., Ukena K. and Tsutsui K. (2001) Brain Res. 899, 112-122
Nazor et al. (2008) Protein Engineering, Design & Selection; 21(1): 29-35
Hwang and Kim (2004) Enzyme and Microbial Technology; 34: 429 - 436
Kaulmann et al. (2007) Enzyme and Microbial Technology; 41: 628-637
Yun et al. (2005) Applied and Environmental microbiology; 4220-4224
Baffert, C., et al. J. Am. Chem. Soc. 2011, 133, 2096-2099
Barker H.A., 1949, J. Biol. Chem. 180: 1085-1093
Bartsch, R.G., 1961, Archives of Biochemistry and biophysics, 92: 122-132
Bornstein B. T., et al., 1948 J Bact, 55:223
Bornstein B. T., et al., 1948 J Biol Chem, 172: 659
Brioukhanov, 2007, Applied Biochemistry and Microbiology, 43 (6): 567-582
Chowdhury N.P., 2014, J.Biol.Chem, 289(8):5145-57
Cotter, J.L (2009) Enzyme and Microbial Technology 44 281-288,
Ding H. et al, 2010, Bioresour Technol, 101(24):9550-9
Drake et al., 2004. Strict and Facultative Anaerobes: Medical and Environmental Aspects. pp. 251-281, Horizon Scientific Press, United Kingdom
Drake & Kusel, 2005 Acetogenic clostridia. In: Dürre, P. (ed.), Handbook on Clostridia, pp. 719-746. CRC Press, Boca Raton, Florida.
Drake et al., 2006, Acetogenic prokaryotes. In: Balows A, Trüper HG, Dworkin M, Harder W and Gerhardt, P et al (ed) American Society for Microbiology, Washington, DC. p. 248-277
Fuchs G., Schlegel H.-G. (2007) Allgemeine Mikrobiologie, Georg Thieme Verlag, Stuttgart.
Henstra A.M., 2007 Current Opinion in Biotechnology, 18:200-206
Hillmer P., 1972; FEBS Letters; 21(3):351-354
Imlay 2006, Molecular Microbiology, 59(4);1073-1082
Koch, AL. 1994. "Growth Measurement" IN: Methods for General and Molecular Bacteriology
Köpke Michael 2009, Dissertation zur Erlangung des Doktorgrades Dr. rer. nat. der Fakultät für Naturwissenschaften der Universität Ulm
Lan, E.I., Energy Environ. Sci., 6:2672
Li, F., 2008, Journal of Bacteriology, 190 (3): 843-850
Lurz R., 1979; Arch Microbiol; 120: 255-262
Madan, V.K., 1972, Eur. J. Biochem.,32;51-56
Najafpour. G., 2006 Enzyme and Microbial Technology 38 (2006) 223-228
Perez, J.M. et al., Biotechnology and Bioengineering, 2012, Vol. xxx, No. xxx
Schleifer KH (eds). The Prokaryotes, 3rd edn. Springer: New York, pp 354-420.
Shuler ML, Kargi F. 1992. Bioprocess Engineering. Prentice Hall, Englewood Cliffs, NJ.
Sliwkowski M.X., 1984, Analytical Biochemistry, 141:344-347
Smith L.T., 1976, Analytical biochemistry, 95:2-7
Smith L.T., 1980, Archives of biochemistry and biophysics, 203 (2): 663-675
Stadtman E.R., 1950, Fed. Proc., 9, 233
Stadtman E.R., 1953, J Biol Chem;202(2):873-90
Steinbusch, 2011, Energy Environ. Sci., 4, 216-224
Thauer, R. K., et al., Eur.K.Biochem., 1974, 42, 447-452
Van Eerten-Jansen, M. C. A. A, 2013, ACS Sustainable Chemistry & Engineering 1 (5), 513-518
Wang S, 2010, Journal of Bacteriology, 192 (19): 5115-5123
Winzer K. et al., 1997 Micrbiology 143:3279-3286
Wood, 1991 Life with CO or CO2 and H2 as a source of carbon and energy. FASEB J. 5:156-163
Younesi H. et al. Biochemical Engineering Journal 27 (2005) 110-119
Zhang Y, 2013, Bioprocess Biosyst Eng; 36(12):1897-1904
U.S. 2007/0275447, U.S. 2008/0057554, WO 98/00558, WO 00/68407
WO2005/111202, WO2007/077041, WO2007/012078 and WO2007/135188

## Claims

1. A microbial cell capable of producing at least one acetylhomoserine from 4-acetyl-butyric acid and/or ester thereof, wherein the cell is genetically modified to comprise
- at least a first genetic mutation that increases the expression relative to the wild type cell of an enzyme E₁ capable of catalysing the hydroxylation of 4-acetyl-butyric acid and/or ester thereof to an alpha-hydroxy acetyl butanone, and
- at least a second genetic mutation that increases the expression relative to the wild type cell of enzyme E₂ capable of catalysing the amination of the alpha-hydroxy acetyl butanone to the acetylhomoserine.

2. The cell according to claim 1, wherein the enzyme E₁ is cytochrome P450 BM3 (CYP102A1).

3. The cell according to either claim 1 or 2, wherein the enzyme E₂ is selected from the group consisting of leucine dehydrogenase and alpha transaminase.

4. The cell according to any one of the preceding claims, wherein E₁ comprises 60% sequence identity with SEQ ID NO: 1.

5. The cell according to any one of the preceding claims, wherein the cell further comprises at least a third genetic mutation that increases the expression relative to the wild type cell of at least one enzyme (E₅) capable of NAD(P)H regeneration.

6. The cell according to claim 5, wherein the enzyme (E₅) is selected from the group consisting of glucose dehydrogenase, phosphite dehydrogenase and formate dehydrogenase.

7. The cell according to any one of the preceding claims, wherein the cell is genetically modified to comprise at least a further genetic mutation that increases the expression relative to the wild type cell of an enzyme E₁₀, an alkane oxidase capable of catalysing the oxidation of butylacetate to 4-acetyl-butyric acid and/or ester thereof.

8. The cell according to claim 7, wherein enzyme E₁₀ is selected from the group consisting of rubredoxin-dependent alkane oxidase (EC 1.14.15.3), cytochrome P450 enzyme (EC 1.14.x.x), xylene monooxygenase (EC: 1.14.13.-; EC: 1.18.1.3) and methane monoxygenase (EC 1.14.13.25; EC 1.14.18.3).

9. A method of producing acetylhomoserine from at least one carbon source, the method comprising a step of contacting an enzyme E₁ with 4-acetyl-butyric acid and/or ester thereof, wherein the enzyme E₁ is capable of catalysing the hydroxylation of 4-acetyl-butyric acid and/or ester thereof to an alpha-hydroxy acetyl butanone.

10. The method according to claim 9, wherein the enzyme E₁ is cytochrome P450 BM3 (CYP102A1).

11. A method of producing acetylhomoserine from 4-acetyl-butyric acid and/or ester thereof, the method comprising:
- contacting the cell according to any one of claims 1 to 6 with a medium comprising 4-acetyl-butyric acid and/or ester thereof.

12. A method of producing acetylhomoserine from butylacetate, the method comprising:
- contacting the cell according to either one of claims 7 or 8 with a medium comprising butylacetate.

13. A method of producing acetylhomoserine from a carbon source, the method comprising the steps of:
(i) contacting a reaction mixture comprising a first and a second microorganism with the carbon source, wherein
- the first microorganism is an acetogenic microorganism capable of converting the carbon source to acetate and/or ethanol;
- the second microorganism is capable of carrying out the ethanol carboxylate fermentation pathway and convert the acetate and/or ethanol to form butyric acid; and
- the first microorganism is further capable of converting the butyric acid to butanol;
(ii) esterifying the butanol to form butylacetate;
(iii) contacting the butylacetate with at least one enzyme E₁₀, an alkane oxidase that is capable of oxidising butylacetate to form 4-acetyl butyric acid and/or ester thereof; and
(iv) contacting the 4-acetyl butyric acid and/or ester thereof with a cell according to any of the claims 1 to 8.

14. The method according to claim 13, wherein the carbon source comprises CO and/or CO₂.

15. The method according to either claim 13 or 14, wherein wherein
- the first microorganism is selected from the group consisting of *Clostridium autothenogenum DSMZ 19630, Clostridium ragsdahlei ATCC no. BAA-622, Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum ATCC* 33266, *Clostridium formicoaceticum, Clostridium butyricum, Lactobacillus delbrukii, Propionibacterium acidoproprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium Ijungdahlii, Clostridium ATCC 29797* and *Clostridium carboxidivorans;* and/or
- the second microorganism is selected from the group consisting of *Clostridium kluyveri,* and C. *Carboxidivorans.*


SEQUENCE LISTING

<110>  Evonik Industries AG

<120>  METHIONINE PRODUCTION

<130>  201500052

<160>  9

<170>  PatentIn version 3.5

<210>  1
<211>  1049
<212>  PRT
<213>  Bacillus megaterium

<400>  1

<210>  2
<211>  3147
<212>  DNA
<213>  Bacillus megaterium

<400>  2
atgacaatta aagaaatgcc tcagccaaaa acgtttggag agcttaaaaa tttaccgtta      60

ttaaacacag ataaaccggt tcaagctttg atgaaaattg cggatgaatt aggagaaatc     120

tttaaattcg aggcgcctgg tcgtgtaacg cgctacttat caagtcagcg tctaattaaa     180

gaagcatgcg atgaatcacg ctttgataaa aacttaagtc aagcgcttaa atttgtacgt     240

gattttgcag gagacgggtt atttacaagc tggacgcatg aaaaaaattg gaaaaaagcg     300

cataatatct tacttccaag cttcagtcag caggcaatga aaggctatca tgcgatgatg     360

gtcgatatcg ccgtgcagct tgttcaaaag tgggagcgtc taaatgcaga tgagcatatt     420

gaagtaccgg aagacatgac acgtttaacg cttgatacaa ttggtctttg cggctttaac     480

tatcgcttta acagctttta ccgagatcag cctcatccat ttattacaag tatggtccgt     540

gcactggatg aagcaatgaa caagctgcag cgagcaaatc cagacgaccc agcttatgat     600

gaaaacaagc gccagtttca agaagatatc aaggtgatga acgacctagt agataaaatt     660

attgcagatc gcaaagcaag cggtgaacaa agcgatgatt tattaacgca tatgctaaac     720

ggaaaagatc cagaaacggg tgagccgctt gatgacgaga acattcgcta tcaaattatt     780

acattcttaa ttgcgggaca cgaaacaaca agtggtcttt tatcatttgc gctgtatttc 840
ttagtgaaaa atccacatgt attacaaaaa gcagcagaag aagcagcacg agttctagta 900
gatcctgttc caagctacaa acaagtcaaa cagcttaaat atgtcggcat ggtcttaaac 960
gaagcgctgc gcttatggcc aactgctcct gcgttttccc tatatgcaaa agaagatacg 1020
gtgcttggag gagaatatcc tttagaaaaa ggcgacgaac taatggttct gattcctcag 1080
cttcaccgtg ataaaacaat ttggggagac gatgtggaag agttccgtcc agagcgtttt 1140
gaaaatccaa gtgcgattcc gcagcatgcg tttaaaccgt ttggaaacgg tcagcgtgcg 1200
tgtatcggtc agcagttcgc tcttcatgaa gcaacgctgg tacttggtat gatgctaaaa 1260
cactttgact ttgaagatca tacaaactac gagctggata ttaaagaaac tttaacgtta 1320
aaacctgaag gctttgtggt aaaagcaaaa tcgaaaaaaa ttccgcttgg cggtattcct 1380
tcacctagca ctgaacagtc tgctaaaaaa gtacgcaaaa aggcagaaaa cgctcataat 1440
acgccgctgc ttgtgctata cggttcaaat atgggaacag ctgaaggaac ggcgcgtgat 1500
ttagcagata ttgcaatgag caaaggattt gcaccgcagg tcgcaacgct tgattcacac 1560
gccggaaatc ttccgcgcga aggagctgta ttaattgtaa cggcgtctta taacggtcat 1620
ccgcctgata acgcaaagca atttgtcgac tggttagacc aagcgtctgc tgatgaagta 1680
aaaggcgttc gctactccgt atttggatgc ggcgataaaa actgggctac tacgtatcaa 1740
aaagtgcctg cttttatcga tgaaacgctt gccgctaaag gggcagaaaa catcgctgac 1800
cgcggtgaag cagatgcaag cgacgacttt gaaggcacat atgaagaatg gcgtgaacat 1860
atgtggagtg acgtagcagc ctactttaac ctcgacattg aaaacagtga agataataaa 1920
tctactcttt cacttcaatt tgtcgacagc gccgcggata tgccgcttgc gaaaatgcac 1980
ggtgcgtttt caacgaacgt cgtagcaagc aaagaacttc aacagccagg cagtgcacga 2040
agcacgcgac atcttgaaat tgaacttcca aaagaagctt cttatcaaga aggagatcat 2100
ttaggtgtta ttcctcgcaa ctatgaagga atagtaaacc gtgtaacagc aaggttcggc 2160
ctagatgcat cacagcaaat ccgtctggaa gcagaagaag aaaaattagc tcatttgcca 2220
ctcgctaaaa cagtatccgt agaagagctt ctgcaatacg tggagcttca agatcctgtt 2280
acgcgcacgc agcttcgcgc aatggctgct aaaacggtct gcccgccgca taaagtagag 2340
cttgaagcct tgcttgaaaa gcaagcctac aaagaacaag tgctggcaaa acgtttaaca 2400
atgcttgaac tgcttgaaaa atacccggcg tgtgaaatga aattcagcga atttatcgcc 2460
cttctgccaa gcatacgccc gcgctattac tcgatttctt catcacctcg tgtcgatgaa 2520
aaacaagcaa gcatcacggt cagcgttgtc tcaggagaag cgtggagcgg atatggagaa 2580
tataaaggaa ttgcgtcgaa ctatcttgcc gagctgcaag aaggagatac gattacgtgc 2640
tttatttcca caccgcagtc agaatttacg ctgccaaaag accctgaaac gccgcttatc 2700

atggtcggac cgggaacagg cgtcgcgccg tttagaggct ttgtgcaggc gcgcaaacag 2760

ctaaaagaac aaggacagtc acttggagaa gcacatttat acttcggctg ccgttcacct 2820

catgaagact atctgtatca agaagagctt gaaaacgccc aaagcgaagg catcattacg 2880

cttcataccg ctttttctcg catgccaaat cagccgaaaa catacgttca gcacgtaatg 2940

gaacaagacg gcaagaaatt gattgaactt cttgatcaag gagcgcactt ctatatttgc 3000

ggagacggaa gccaaatggc acctgccgtt gaagcaacgc ttatgaaaag ctatgctgac 3060

gttcaccaag tgagtgaagc agacgctcgc ttatggctgc agcagctaga agaaaaaggc 3120

cgatacgcaa aagacgtgtg ggctggg 3147

<210> 3
<211> 310
<212> PRT
<213> Artificial Sequence

<220>
<223> L-HIC-DH

<400> 3

Met Ala Arg Lys Ile Gly Ile Ile Gly Leu Gly Asn Val Gly Ala Ala
1               5                   10                  15

Val Ala His Gly Leu Ile Ala Gln Gly Val Ala Asp Asp Tyr Val Phe
            20                  25                  30

Ile Asp Ala Asn Glu Ala Lys Val Lys Ala Asp Gln Ile Asp Phe Gln
        35                  40                  45

Asp Ala Met Ala Asn Leu Glu Ala His Gly Asn Ile Val Ile Asn Asp
    50                  55                  60

Trp Ala Ala Leu Ala Asp Ala Asp Val Val Ile Ser Thr Leu Gly Asn
65                  70                  75                  80

Ile Lys Leu Gln Gln Asp Asn Pro Thr Gly Asp Arg Phe Ala Glu Leu
                85                  90                  95

Lys Phe Thr Ser Ser Met Val Gln Ser Val Gly Thr Asn Leu Lys Glu
            100                 105                 110

Ser Gly Phe His Gly Val Leu Val Val Ile Ser Asn Pro Val Asp Val
        115                 120                 125

Ile Thr Ala Leu Phe Gln His Val Thr Gly Phe Pro Ala His Lys Val
    130                 135                 140

Ile Gly Thr Gly Thr Leu Leu Asp Thr Ala Arg Met Gln Arg Ala Val
145                 150                 155                 160

Gly Glu Ala Phe Asp Leu Asp Pro Arg Ser Val Ser Gly Tyr Asn Leu
                165                 170                 175

Gly Glu His Gly Asn Ser Gln Phe Val Ala Trp Ser Thr Val Arg Val
            180                 185                 190

Met Gly Gln Pro Ile Val Thr Leu Ala Asp Ala Gly Asp Ile Asp Leu
        195                 200                 205

Ala Ala Ile Glu Glu Glu Ala Arg Lys Gly Gly Phe Thr Val Leu Asn
    210                 215                 220

Gly Lys Gly Tyr Thr Ser Tyr Gly Val Ala Thr Ser Ala Ile Arg Ile
225                 230                 235                 240

Ala Lys Ala Val Met Ala Asp Ala His Ala Glu Leu Val Val Ser Asn
                245                 250                 255

Arg Arg Asp Asp Met Gly Met Tyr Leu Ser Tyr Pro Ala Ile Ile Gly
            260                 265                 270

Arg Asp Gly Val Leu Ala Glu Thr Thr Leu Asp Leu Thr Thr Asp Glu
        275                 280                 285

Gln Glu Lys Leu Leu Gln Ser Arg Asp Tyr Ile Gln Gln Arg Phe Asp
    290                 295                 300

Glu Ile Val Asp Thr Leu
305                 310

<210>  4
<211>  429
<212>  PRT
<213>  Artificial Sequence

<220>
<223>  Leucine-DH

<400>  4

Met Glu Leu Phe Lys Tyr Met Glu Thr Tyr Asp Tyr Glu Gln Val Leu
1               5                   10                  15

Phe Cys Gln Asp Lys Glu Ser Gly Leu Lys Ala Ile Ile Ala Ile His
            20                  25                  30

Asp Thr Thr Leu Gly Pro Ala Leu Gly Gly Thr Arg Met Trp Met Tyr
        35                  40                  45

Asn Ser Glu Glu Glu Ala Leu Glu Asp Ala Leu Arg Leu Ala Arg Gly
    50                  55                  60

Met Thr Tyr Ser Asn Ala Ala Ala Gly Leu Asn Leu Gly Gly Gly Lys
65                  70                  75                  80

Thr Val Ile Ile Gly Asp Pro Arg Lys Asp Lys Asn Glu Ala Met Phe
                85                  90                  95

Arg Ala Phe Gly Arg Phe Ile Gln Gly Leu Asn Gly Arg Tyr Ile Thr
            100                 105                 110

Ala Val Asp Val Gly Thr Thr Val Ala Asp Met Asp Ile Ile Tyr Gln
        115                 120                 125

Glu Thr Asp Tyr Val Thr Gly Ile Ser Pro Glu Phe Gly Ser Ser Gly
    130                 135                 140

Asn Pro Ser Pro Ala Thr Ala Tyr Gly Val Tyr Arg Gly Met Lys Ala
145                 150                 155                 160

Ala Ala Lys Glu Ala Phe Gly Ser Asp Ser Leu Glu Gly Lys Val Val
                165                 170                 175

Ala Val Gln Gly Val Gly Asn Val Ala Tyr His Leu Cys Arg His Leu
            180                 185                 190

His Glu Glu Gly Ala Lys Leu Ile Val Thr Asp Ile Asn Lys Glu Val
        195                 200                 205

Val Ala Arg Ala Val Glu Glu Phe Gly Ala Lys Ala Val Asp Pro Asn
    210                 215                 220

Asp Ile Tyr Gly Val Glu Cys Asp Ile Phe Ala Pro Cys Ala Leu Gly
225                 230                 235                 240

Gly Ile Ile Asn Asp Gln Thr Ile Pro Gln Leu Lys Ala Lys Val Ile
                245                 250                 255

Ala Gly Ser Ala Asp Leu Gln Leu Lys Glu Pro Arg His Gly Asp Ile
            260                 265                 270

Ile His Glu Met Gly Ile Val Tyr Ala Pro Asp Tyr Val Ile Asn Ala
        275                 280                 285

Gly Gly Cys Ile Asn Val Ala Asp Glu Leu Tyr Gly Tyr Asn Arg Glu
    290                 295                 300

Arg Ala Met Lys Lys Ile Glu Gln Ile Tyr Asp Asn Ile Glu Lys Val
305                 310                 315                 320

Phe Ala Ile Ala Lys Arg Asp Asn Ile Pro Thr Tyr Val Ala Ala Asp
                325                 330                 335

Arg Met Ala Glu Glu Arg Ile Glu Thr Met Arg Lys Ala Ala Ser Gln
            340                 345                 350

Phe Leu Gln Asn Gly His His Ile Leu Ser Arg Arg Pro Arg Pro Leu
        355                 360                 365

Thr Ala Ala Arg Ala Gly Leu Arg Arg Ala Asp Asp Gly Gly Thr Thr
    370                 375                 380

Thr Met Gln Glu Gln Lys Phe Arg Ile Leu Thr Ile Asn Pro Gly Ser
385                 390                 395                 400

Thr Ser Thr Lys Ile Gly Val Phe Glu Asn Glu Arg Ala Ile Ala Ser
                405                 410                 415

Lys Lys Arg Ser Ala Thr Arg Ala Gly Ala Ser Ala Ile
            420                 425

<210>  5
<211>  333
<212>  PRT
<213>  Artificial Sequence

<220>
<223>  D-HIC-DH

<400>  5

Met Lys Ile Ile Ala Tyr Gly Ala Arg Val Asp Glu Ile Gln Tyr Phe
1               5                   10                  15

Lys Gln Trp Ala Lys Asp Thr Gly Asn Thr Leu Glu Tyr His Thr Glu
            20                  25                  30

Phe Leu Asp Glu Asn Thr Val Glu Trp Ala Lys Gly Phe Asp Gly Ile
        35                  40                  45

Asn Ser Leu Gln Thr Thr Pro Tyr Ala Ala Gly Val Phe Glu Lys Met
    50                  55                  60

His Ala Tyr Gly Ile Lys Phe Leu Thr Ile Arg Asn Val Gly Thr Asp
65                  70                  75                  80

Asn Ile Asp Met Thr Ala Met Lys Gln Tyr Gly Ile Arg Leu Ser Asn
                85                  90                  95

Val Pro Ala Tyr Ser Pro Ala Ala Ile Ala Glu Phe Ala Leu Thr Asp
            100                 105                 110

Thr Leu Tyr Leu Leu Arg Asn Met Gly Lys Val Gln Ala Gln Leu Gln
        115                 120                 125

Ala Gly Asp Tyr Glu Lys Ala Gly Thr Phe Ile Gly Lys Glu Leu Gly
    130                 135                 140

Gln Gln Thr Val Gly Val Met Gly Thr Gly His Ile Gly Gln Val Ala
145                 150                 155                 160

Ile Lys Leu Phe Lys Gly Phe Gly Ala Lys Val Ile Ala Tyr Asp Pro
                165                 170                 175

Tyr Pro Met Lys Gly Asp His Pro Asp Phe Asp Tyr Val Ser Leu Glu
            180                 185                 190

Asp Leu Phe Lys Gln Ser Asp Val Ile Asp Leu His Val Pro Gly Ile
        195                 200                 205

Glu Gln Asn Thr His Ile Ile Asn Glu Ala Ala Phe Asn Leu Met Lys
    210                 215                 220

Pro Gly Ala Ile Val Ile Asn Thr Ala Arg Pro Asn Leu Ile Asp Thr
225                 230                 235                 240

Gln Ala Met Leu Ser Asn Leu Lys Ser Gly Lys Leu Ala Gly Val Gly
                245                 250                 255

Ile Asp Thr Tyr Glu Tyr Glu Thr Glu Asp Leu Leu Asn Leu Ala Lys
            260                 265                 270

His Gly Ser Phe Lys Asp Pro Leu Trp Asp Glu Leu Leu Gly Met Pro
        275                 280                 285

Asn Val Val Leu Ser Pro His Ile Ala Tyr Tyr Thr Glu Thr Ala Val
    290                 295                 300

His Asn Met Val Tyr Phe Ser Leu Gln His Leu Val Asp Phe Leu Thr
305                 310                 315                 320

Lys Gly Glu Thr Ser Thr Glu Val Thr Gly Pro Ala Lys
                325                 330

<210>  6
<211>  42
<212>  DNA
<213>  Artificial Sequence

<220>
<223>  primer: R47/Y51 Fw

<220>
<221>  misc_feature
<222>  (14)..(15)
<223>  n is a, c, g, or t

<220>
<221>  misc_feature
<222>  (26)..(27)
<223>  n is a, c, g, or t

<400>  6
cgaggcgcct ggtnnkgtaa cgcgcnnktt atcaagtcag cg                          42

<210>  7
<211>  42
<212>  DNA
<213>  Artificial Sequence

<220>
<223>  primer: R47/Y51 Rv

<220>
<221>  misc_feature
<222>  (16)..(17)
<223>  n is a, c, g, or t

<220>
<221>  misc_feature
<222>  (28)..(29)
<223>  n is a, c, g, or t

<400>  7
cgctgacttg ataamnngcg cgttacmnna ccaggcgcct cg                          42

<210>  8
<211>  27
<212>  DNA
<213>  Artificial Sequence

<220>
<223>  primer: I401 Fw

<220>
<221>  misc_feature
<222>  (13)..(14)

<223>  n is a, c, g, or t

<400>  8
cagcgtgcgt gtnnkggtca gcagttc                                          27

<210>  9
<211>  27
<212>  DNA
<213>  Artificial Sequence

<220>
<223>  primer: I401 Rw

<220>
<221>  misc_feature
<222>  (14)..(15)
<223>  n is a, c, g, or t

<400>  9
gaactgctga ccmnnacacg cacgctg                                          27